# EUROPEAN PATENT APPLICATION

(11) **EP 1 327 883 A2**
(43) Date of publication of application: **16.07.2003**
(21) Application number: 02000615.1
(22) Date of filing: 10.01.2002
(51) Int. Cl.: G01N 33/50, G01N 33/68, C12Q 1/68

(54) **Combined metabolomic, proteomic and transcriptomic analysis from one, single sample and suitable statistical evaluation of data**

(71) Applicant: Max-Planck-Gesellschaft zur Förderung der Wissenschaften e.V., Berlin (DE)
(72) Inventor: Fiehn, Oliver, 10407 Berlin (DE); Weckwerth, Wolfram, 14089 Berlin (DE)
(74) Representative: VOSSIUS & PARTNER

(57) **Abstract**

Disclosed is a method for providing data useful for quantitatively analyzing metabolites, proteins and/or RNA in a biological source material, whereby said analysis involves suitable statistical evaluation and correlation analysis on the data obtained, preferably also including network analysis, said method being characterized by the step of extracting, identifying and quantifying at least two compound classes of the group consisting of metabolites, proteins and RNA from at least one sample from said biological source material, wherein the compounds of said at least two classes are each determined from one sample. Furthermore disclosed is a corresponding method for quantitatively analyzing metabolites, proteins and/or RNA in a biological source material comprising providing data on metabolites, proteins and/or RNA, performing suitable statistical evaluation and correlation analysis on the data obtained; and optionally further performing a network analysis. Also disclosed is the use of a mixture of solvents suitable for extracting RNA, proteins and metabolites from a biological sample for extracting metabolites, and optionally also proteins and/or RNA from a biological sample in order to perform metabolite profiling.

## Description

The present invention relates to a method for providing data useful for quantitatively analyzing metabolites, proteins and/or RNA in a biological source material, whereby said analysis involves suitable statistical evaluation and correlation analysis on the data obtained, preferably also including network analysis, said method being characterized by the step of extracting, identifying and quantifying at least two compound classes of the group consisting of metabolites, proteins and RNA from at least one sample from said biological source material, wherein the compounds of said at least two classes are each determined from one sample. The invention furthermore relates to a corresponding method for quantitatively analyzing metabolites, proteins and/or RNA in a biological source material comprising providing data on metabolites, proteins and/or RNA, performing suitable statistical evaluation and correlation analysis on the data obtained; and optionally further performing a network analysis. Moreover, the invention relates to the use of a mixture of solvents suitable for extracting RNA, proteins and metabolites from a biological sample for extracting metabolites, and optionally also proteins and/or RNA from a biological sample in order to perform metabolite profiling.

In the post-genomic era, a functional assignment of genes is getting more and important that goes beyond simple homology search and rough estimations of biochemical and biological roles. Instead, the scientific community tends towards a more comprehensive understanding of biology. Accordingly, the function of a gene is now regarded as depending on developmental and environmental changes as well as on the actual expression level of other genes rather than a simple, linear cause-event relationship. This change in paradigm will necessarily lead to the need to experimentally describe the state of biological tissues in depth on different levels, i.e. not only on the level of transcripts or protein expression, but also on the metabolite level and the interrelatedness of these levels. For example, Fiehn (Comp. Funct. Genom. 2 (2001), 155-168) suggests a combination of results from in depth characterization of genetically altered organisms using transcriptomics, proteomics and metabolomics in order to more comprehensively understand living organisms. Metabolites are the end products of cellular regulatory processes, and their levels can be regarded as the ultimate response of biological systems to genetic or environmental changes. In parallel to the terms "transcriptome" and "proteome", the set of metabolites synthesized by a biological system compose its "metabolome".
In the growing fields of transcriptomics, proteomics and metabolomics, there is a rapidly ongoing progress in improving the techniques necessary for collecting data and for processing the data obtained. Current strategies and limitations for the quantitative analysis of cellular responses at all of the three levels mRNA, proteins, and metabolites have been recently summarized in a short review by Fiehn (Curr. Opin. Biotechnol. 12 (2001), 82-86) including thoughts on database requirements and informatic tools.
Today, transcriptomic approaches seem to give the best coverage of genome level responses. However, due to limitations in analytical precision and high costs, few transcriptomic studies adequately meet rigid statistical requirements. There are several prominent analytical approaches used for transcript profiling: microarray-based approaches (cDNAs or oligonucleotides) (e.g. reviewed in Granjeaud, Bioessays 21 (1999), 781-790); sequencing-based approaches, such as serial analysis of gene expression (SAGE) (Velculescu, Science 270 (1995), 484-487) or massively parallel signature sequencing (MPSS) (Brenner, Nat. Biotechnol. 18 (2000), 630-634); and differential-display-based approaches, such as arbitrarily primed (AP) PCR (Welsh, Nucl. Acids Res. 20 (1992), 4965-4970) and cDNA-amplified fragment length polymorphism (AFLP) (Bachem, Plant J. 9 (1996), 745-753). Of all of these, microarray technology has received the most widespread interest. This technique has been used extensively in yeast and human work, and thousands of expression profiles have been examined (e.g. Hughes, Cell 102 (2000), 109-126).
For proteomic approaches, two-dimensional gel electrophoresis (2DE) is well established in many biological laboratories (Thiellement, Electrophoresis 20 (1999), 2013-2026) and is comparatively inexpensive. However, if the full set of proteins separated by 2DE gel is to be identified, highly automated systems are needed for cutting spots, digesting proteins, and analyzing peptides using mass spectrometry. Capillary isoelectric focusing is an alternative to 2DE that can be directly coupled to ion cyclotron resonance mass spectrometers (Fourier-transform mass spectrometry, FT-MS) for the analysis of both crude protein mixtures (Jensen, Electrophoresis 21 (2000), 1372-1380) and complex peptide digests (Gao, J. Microcolumn 12 (2000), 383-390). Further advances have been made with approaches where liquid chromatographic (LC) separation has been combined with mass spectrometrical detection (MS). For instance, LC/LC/MS techniques allow more sensitive and rapid separations of complex peptide mixtures when cation exchange is coupled to reversed phase LC columns. This strategy has been shown to be superior in speed, robustness and protein coverage, when compared with 2DE analysis (Link, Nat. Biotechnol. 17 (1999), 676-682). According to a rather new technique, quantification of protein abundances can be performed using isotope coded affinity tags (ICAT) with precisions as accurate as 12% relative standard deviations (Gygi, Nat. Biotechnol. 17 (1999), 1112-1118). But to date, this technique has not been utilized for proteomic studies that go beyond one-to-one comparative experiments.
Compared to transcriptomic and proteomic approaches, analytical techniques for metabolite detection and quantification are far more robust and mature. Analytical precisions may be below 1% relative standard deviations, and dynamic ranges may exceed four orders of magnitude. For instance in plant research, mass spectrometry has been used for decades to determine metabolic target compounds, but only recently has the idea been pursued of expanding the list of targets in order to profile a limited number of primary metabolites. Moreover, estimates of the total number of metabolites different members of the plant kingdom synthesise, that is estimated of plant metabolome sizes, range from 90,000-200,000. To determine comprehensive metabolomes, therefore, one must cope with the sheer complexity of mass spectra found in chromatograms and apply new technologies for efficient *de novo* compound identification. Most recently, high-througput profiling of metabolic snapshots has been demonstrated for the first time in the context of plant functional genomics (Fiehn, Nat. Biotechnol. 18 (2000), 1157-1161). Profiles of 326 metabolites of two Arabidopsis ecotypes were compared with two single gene mutants. Cluster analysis revealed distinct "metabolic phenotypes" for each of the four genotypes.
On the other hand, evaluation of the profiling data from transcriptome, proteome and metabolome by bioinformatics is also currently in progress. On the basis of the assumption that large sets of genes and proteins follow synchronized patterns, attempts at taming profiling data have been made using clustering algorithms that group raw data in an unbiased way (Eisen, Proc. Natl. Acad. Sci. USA 95 (1998), 14863-14868). Clustering allows genes, proteins or metabolites to be grouped according to their profiles.

All clustering methods used so far, however, have significant drawbacks (Bittner, Nat. Genet. 22 (1999), 213-215) that make them unsuitable for detecting complex relationships in data networks. The inability to detect non-linear correlations is one such limitation. A new way to cover similarities and correlation of expression profiles based on mutual informational entropy has been proposed by Butte (Proc. Natl. Acad. Sci. USA 97 (2000), 12182-12186). Until recently, all clustering methods also lacked a means by which to integrate statistics. Each gene or experiment was mapped into n-dimensional expression space without covering the fact that the sharp point within this space is merely a probability cloud. Hughes (Cell 102 (2000), 109-126) made an attempt to include statistics in their clustering methods so as to see which clusters were trustworthy. In addition, profiling bioinformatics is moving towards methods that try to incorporate as much available knowledge as possible. Recently, two groups used statistical methods to correlate expression profiles with potential promoter sequences (Jensen, Bioinformatics 16 (2000), 326-333; Tavazoie, Nat. Genet. 22 (1999), 281-285). Marcotte (Nature 402 (1999), 83-86) tried to elucidate the functions of unknown ORFs on the basis of five different data sources, including expression profiling data. Brown (Proc. Natl. Acad. Sci USA 97 (2000), 262-267) used a special neuronal network to identify potential gene functions on the basis of the expression profile and the transcriptional patterns of well-annotated genes.

In view of the above outlines, it is evident that the developments in obtaining comprehensive data on the levels of RNA, proteins and metabolites and the attempts to derive therefrom new insights into the complex regulation of gene expression appear to be quite promising, however, also require further improvements.

Thus, the technical problem underlying the present invention is to provide a method that allows it to improve the meaningfulness of correlation data on gene expression and metabolite states of a given biological source material.

This technical problem is solved by the provisions of the embodiments as characterized in the claims.

Accordingly, the present invention relates to a method for providing data useful for quantitatively analyzing metabolites, proteins and/or RNA in a biological source material, whereby said analysis involves suitable statistical evaluation and correlation analysis on the data obtained, preferably also including network analysis,
said method being characterized by the step of extracting, identifying and quantifying at least two compound classes of the group consisting of metabolites, proteins and RNA from at least one sample from said biological source material, wherein the compounds of said at least two classes are each determined from one sample.

The particular achievement of the present invention lies in the fact that at least two of the three compound classes RNA, proteins and metabolites are each extracted and analyzed from one single sample. Accordingly, the phrase "the compounds of said at least two classes are each determined from one sample" means that, for each sample collected in the course of applying the method of the invention, the compounds of all the compound classes analyzed are extracted from one and the same sample.
The method of the present invention may be carried out altogether taking one sample or taking more than one, preferably a multitude of samples. For example, to provide data from one sample may be applicable if corresponding reference data is already available from other samples, e.g. in a database. Data from a series of samples can for instance be useful for investigating a process along a time course, such as a developmental process. On the other hand, samples taken from different genotypes of organisms being at the same developmental stage may likewise deliver useful data, e.g., for characterizing the influence of a particular genotype on gene expression, protein composition and/or metabolite composition.

The method of the present invention represents a clear improvement over prior art techniques for providing data, e.g. for network analyses, since there data on RNA levels, protein levels and/or metabolite states were each sampled individually, i.e. determined in different samples. Thus, aiming at combining data sets from e.g. two compound classes hitherto was hampered by the uncertainty of whether the individual data sets actually represent corresponding cellular states. Since cellular states may vary depending on developmental or environmental conditions or on the genotype, it is often uncertain whether such data sets to be combined are indeed compatible, i.e. so that any correlations measured, e.g. between metabolite data and gene expression data, indeed reflect the actual processes in the cells under investigation. In addition, prior art attempts to combine data from different compound classes may suffer from calibration problems. In particular, these occur when one wants to directly set absolute values from one data set into relation to absolute values from another data set. To do so would require to have control values within each data set that allows it to calibrate the data. These drawbacks, i.e. the uncertainty of potential non-compatibility and calibration problems, are largely overcome or at least substantially reduced by the provisions of the present invention. By deriving the quantitative data on two or three different compound classes each from one single sample, it is guaranteed that the data sets obtained reflect just the same cellular status of the biological source material. Also calibration should present a decisively reduced problem if the data sets are obtained according to the method of the invention. Since the amount of material extracted is the same for each compound class analyzed of one sample and the data on the individual compound classes are derived from the identical cells, it can be expected that absolute values of different compound classes are directly correlatable without need of calibration. Still if, due to a certain way of subsequently extracting the different compounds from one sample, the absolute values finally obtained will not be directly correlatable, the person skilled in the art knows how to relate the values obtained to the amount of starting material extracted so that the absolute values may resume their direct correlatability.

The method of the present invention relates to the provision of quantitative data on at least two compound classes in a biological source material. The term "compound class" relates to a group of compounds which is either metabolites, proteins or RNA. In a preferred embodiment, data on all three compound classes are obtained by said method. Also preferred is the combination of metabolites with either proteins or RNA. The term "metabolite" refers to any substance within a certain biological source material that is non-peptidic and not a nucleic acid molecule. Preferably, the metabolites addressed by the present invention rather have a lower molecular weight, i.e. for instance not more than 4000 Da, preferably not more than 2000 Da, more preferably not more than 1000 Da. Typically, the metabolites to be analyzed belong to the following, however non-limiting list of compounds: carbohydrates (e.g. sugars, oligo- and polysaccharides such as polyglucans as for example starch or polyfructans), sugar alcohols, amines, amino alcohols, aliphatics, aliphatic alcohols, amino acids, lipids, fatty acids, fatty alcohols, organic acids, organic phosphates, organic or anorganic ions, nucleotides, sugar nucleotides, sterols, terpenes, terpenoids, flavons and flavonoids, glucosides, carotenes, carotenoids and cofactors.
The term "proteins" refers to any molecules comprising amino acids connected via peptide bonds that are present in the biological source material.
The term "RNA" refers to any ribonucleic acids that can be present in cells contained in the biological source material. Preferred RNA is mRNA, i.e. transcripts from protein-encoding genes. The present method may also be applied by detecting other RNA species than mRNA such as rRNA, tRNA or viral RNA.
The term "biological source material" means any material being or containing living matter, such as cells, tissues, organs or organisms. The method is not restricted to any taxon. Thus, prokaryotes such as archaebacteria or eubacteria (gram-positive or gram-negative) as well as eukaryotes such as yeasts, fungi, plants (i.e. algae or land plants, in particular flowering plants) or animals as for instance insects or vertebrates, especially mammals, and corresponding cell cultures are within its reach. As a preferred biological source material, plant tissue, e.g. leaves, can be used. The feasibility of the method of the invention has first been demonstrated at Arabidopsis leaf (see Example). Other preferred biological source materials may be the classical objects of genetics, such as Drosophila, E.coli, yeast, especially Saccharomyces cerevisiae or C. elegans where lots of mutants are described and regulatory networks integrating, e.g. metabolites and gene expression data, are promising to reveal new insights into gene regulation, e.g. during development. In a further important aspect, the method of the invention may help to improve drug discovery or cancer research. In this context, corresponding biological source material would for example be derivable from animals such as vertebrates, typically birds or mammals. In connection to this, preferred examples of biological examples may be mammalian, preferably human cell lines or tissue from test animals such as laboratory mice or rats.
The term "sample" encompasses any amount of material taken from the biological source material that is susceptible to the method of the invention. For instance, a sample can be fresh material such as a tissue explant, a body fluid or an aliquot from a bacterial or cell culture, preferably deprived of the culture medium, that may be directly subjected to extraction. On the other hand, samples may also be stored for a certain time period, preferably in a form that prevents destructive action of inherent enzymatic activity, e.g. frozen, for instance in liquid nitrogen, or lyophilized.

Extraction should be carried out so that the compounds one is interested in are dissolved as completely and quantitatively as possible and in a manner that they can later be identified and quantified using appropriate methods. The term "extracting" thereby refers to contacting the material containing the compounds of interest with an extractant (e.g. solvent or mixture of solvents) so that these compounds can be dissolved, followed by separating the solution from the undissolved matter. The person skilled in the art is capable of choosing an appropriate extraction protocol that is suited to isolate the two or three compound classes of interest and which is useful for the biological source material the sample is taken from. In the prior art, methods are described that may be especially useful within the context of the present invention, such as subsequent application of pure solvents or methanol/water mixtures, or other methods such as microwave extraction, ultrasonication, or accelerated solvent extraction.
Usually, it is advantageous to use an extractant that contains or is an organic (i.e. lipophilic) solvent so that the proteins are denatured upon addition of the extractant so that other compounds, i.e. metabolites and/or RNA will not be enzymatically degraded or modified. Stopping of inherent enzymatic activity can also be achieved by freeze clamping, immediate freezing in liquid nitrogen, or by acidic treatments using perchloric or nitric acid (ap Rees and Hill, 1994). Although advantageous for extraction of amines (Bouchereau et al. 2000), acidic treatments may possibly pose problems for many subsequent analytical methods. In a preferred way, frozen samples can be directly extracted by immediately adding organic solvents and applying heat, thereby also inhibiting the recovery of enzymatic activity. Extracting frozen samples that still contain the original amount of water can be advantageous for applications including analysis of metabolites when compared to extracting lyophilized samples, since lyophilisation may potentially lead to the irreversible adsorption of metabolites on cell walls or membranes. In cases where it is desired to distinguish between metabolite levels in different compartments, samples can be lyophilized prior to non-aqueous fractionation methods (Gerhardt and Heldt 1984, Farré et al. 2001). An alternative approach to non-aqueous fractionation is the use of nuclear magnetic resonance analyses (NMR) to distinguish steady state concentrations of metabolites in different compartments in vivo (Roberts 2000). For tissue culture, a cold shock can be used in which the liquids are infused into cold methanol. All devices needed for further sample preparation should then be kept at cold temperatures (Gonzalez et al. 1997). In addition, polar organic solvents like methanol, methanol-water mixtures, or ethanol can be directly added to freshly frozen tissues (Johansen et al. 1996, Streeter and Strimbu 1998), with an additional step of using non-polar solvents such as chloroform to exhaustively extract lipophilic components. In order to enhance the extraction efficiency, additional energy may be put into the system either directly by heat (e.g. 70°C), or by other techniques such as pressurized liquid extraction (Benthin et al. 1999), supercritical fluid extraction (Jarvis and Morgan 1997, Blanch et al. 1999, Castioni et al. 1995), sonication (Sargenti and Vichnewski 2000), subcritical water extraction (Gàmiz-Gracia and de Castro 2000), microwave techniques (Namiesnik and Gorecki 2000), or pervaporation (Starmans and Nijhuis 1996).

In a preferred embodiment of the method of the invention, extracting comprises the steps of:
(a) extracting the metabolites from said sample with at least one solvent or mixture of solvents;
(b) extracting the proteins from the remainder of the sample after step (a);
(c) extracting the RNA from the remainder of the sample after step (a); and
(d) optionally dissolving remaining cellular material contained in said sample.
   This sequence of steps describes the isolation of all three compound classes. If, however, one is interested in data of only two compound classes, these steps may be adapted correspondingly, for instance by omitting step (a) or (b) or (c), depending on whether metabolites, protein or RNA shall not be analyzed. The sequence of steps (a) to (d) is not necessarily chronological. In particular, step (c) may for instance also be carried out before step (b) or simultaneously.
In step (a), the sample is extracted with an extractant that comprises at least a solvent or a mixture of solvents. The extractant may contain additional substances such as antioxidants, detergents or buffering agents. The term "solvent" refers to the common meaning as used in the prior art, while "aqueous solvent" refers to water or an aqueous buffer.

In a particularly preferred embodiment, step (a) is carried out with a mixture of solvents that comprises at least one highly polar solvent, at least one less polar solvent and at least one lipophilic solvent.
The terms "highly polar", "less polar" and "lipophilic" in connection with solvent has a clear meaning to the person skilled in the art. Usually, the polarity of a solvent is defined by reference to the dielectric constant (ε) of the solvent. This quantity is temperature-dependent, often it is given for solvents at 20°C. In connection with the method of the invention, "highly polar" solvents have a dielectric constant in the range from above 35 to 90, preferably from 36 to 81. "Less polar" solvents have a dielectric constant in the range of above 5.5 to 35, preferably from 6 to 33. "Lipophilic" solvents have a dielectric constant in the range from 1.0 to 5.5, preferably 1.8 to 5.0. Typical examples of highly polar solvents are acetonitrile, N,N-dimethylacetamide, N,N-dimethylformamide, dimethyl sulfoxide, glycerol, nitromethane and water. Typical expamples of less polar solvents are acetic acid, acetone, 2-butanol, 1,2-dichloroethane, dichloromethane, ethanol, ethyl acetate, methanol, 1-propanol, 2-propanol, pyridine and tetrahydrofuran. Furthermore, typical examples of lipophilic solvents are benzene, carbon tetrachloride, chloroform, cyclohexane, diethyl ether, 1,4-dioxane, heptane, hexane, pentane, tetrachloroethylene and toluene.
Preferably, the mixture of highly polar, less polar and lipophilic solvents has one phase. Accordingly, the solvents should be chosen so that, at the particular conditions of extraction applied, the mixture of solvents used has one phase. More preferably, the mixture of solvents for use in step (a) comprises water, methanol and chloroform, most preferably these solvents are contained in the mixture in the approximate proportion by volume of 1:2.5:1

In the method of the invention, the temperature under which extraction is carried out can be crucial to successfully extract a broad range of compounds, in particular metabolites. In the experiments underlying the present invention, a cold temperature has been shown to be favorable in this regard. Accordingly, it is preferred that extraction, in particular the above-mentioned step (a) of the preferred extraction scheme, is carried out at a temperature in the range between -60°C and +4°C, more preferably between -40°C and -10°C, still more preferably between -20°C and -13°C and most preferably at -16°C. Practically, extraction should be carried out at a temperature where the solvents used are liquid.

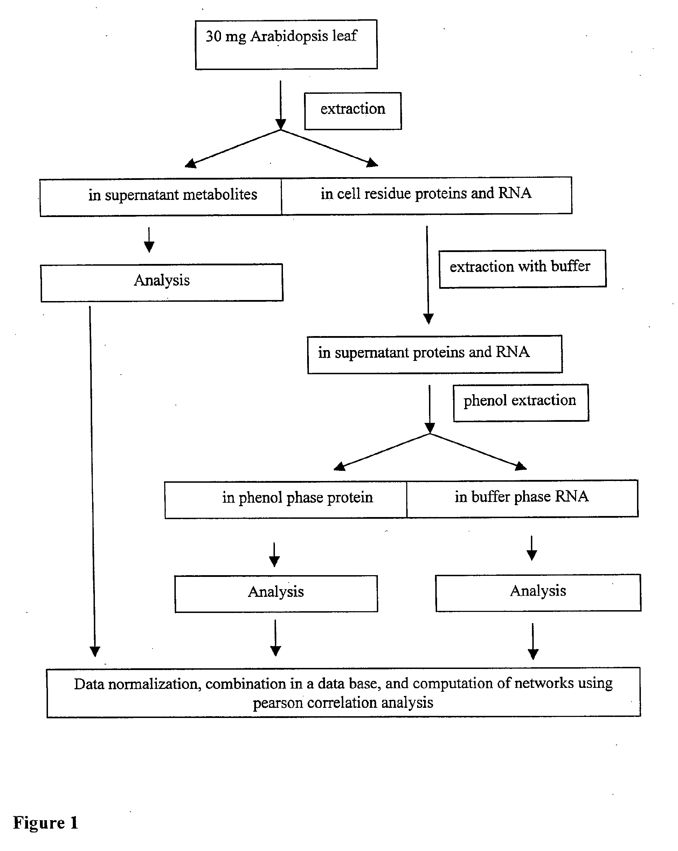

Steps (a) to (d) of the above-described preferred embodiment of the method of the invention can be carried out according to techniques known to the person skilled in the art and described in the literature. In order to illustrate the sequence of the extraction steps, Figure 1 shows a specific scheme containing steps (a) to (c).
Generally, in step (a), the metabolites are extracted from the sample. For this purpose, the cells or tissue may be broken up using appropriate techniques such as homogenization. After incubating the sample material for sufficient time to dissolve the metabolites quantitatively in the extractant, the solution containing the metabolites should be separated from undissolved material (remainder of the sample).
In steps (b) and (c), the proteins and RNA may be extracted from the remainder of the sample after step (a), using a suitable extractant, e.g. a buffered aqueous solution. The resulting extract can then be subjected to a further separation step, wherein proteins are separated from RNA. This can, for instance, be done by phenol extraction, i.e. by adding phenol, mixing and centrifuging with the result that RNA is in the aqueous phase and proteins in the phenol phase. An overview of various RNA isolation techniques is described in Sambrook and Russell (2001) and Gassen and Schrimpf (1999).
Optionally, as step (d), dissolving remaining cellular material contained in the sample may also be performed in order to provide additional compounds for quantitative analysis. Such undissolved material may comprise membranes or cell wall material that can be dissolved using suitable means such as hydrolyzing enzymes. The hydrolysates may for instance be added to the metabolite fraction or may be analyzed individually.

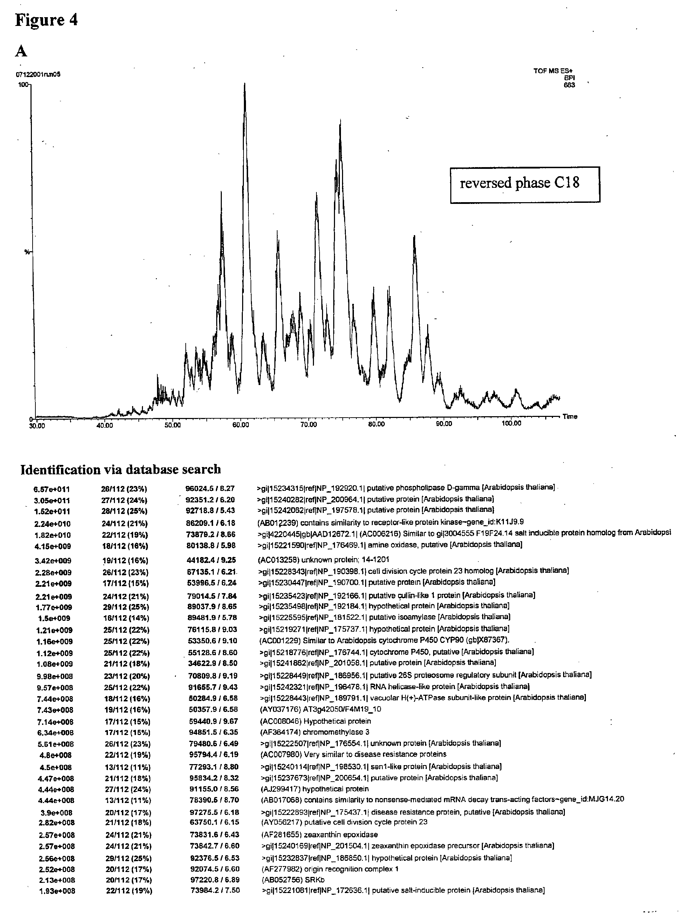

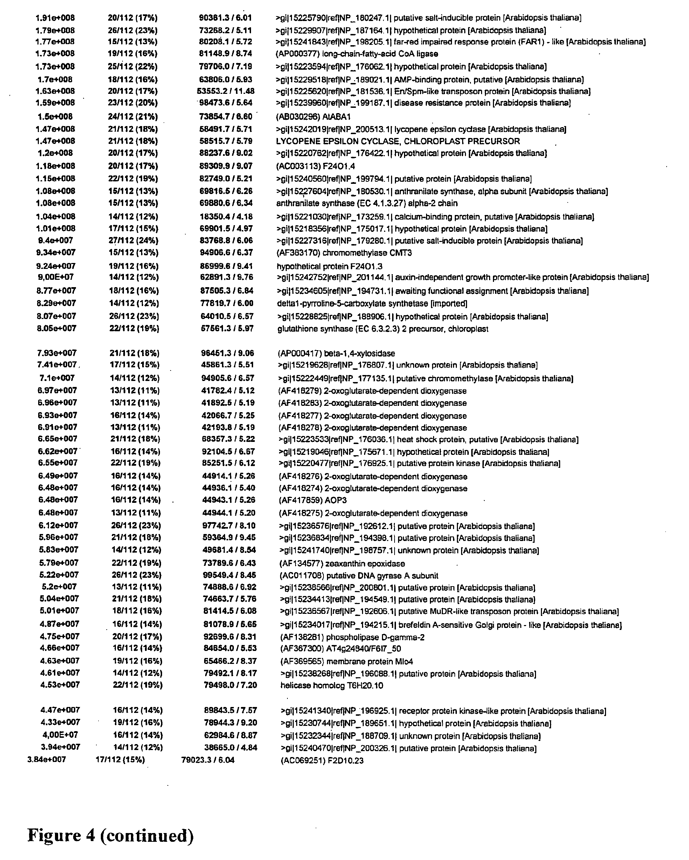

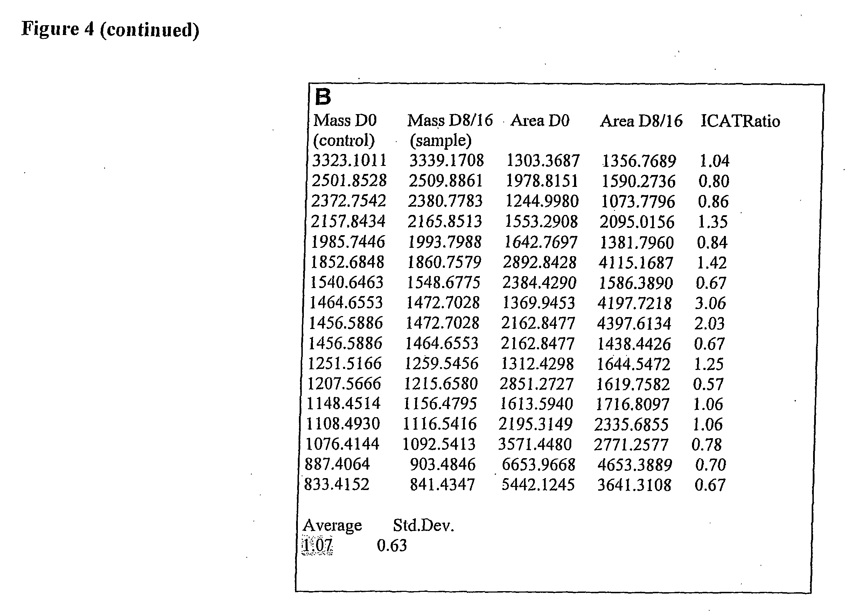

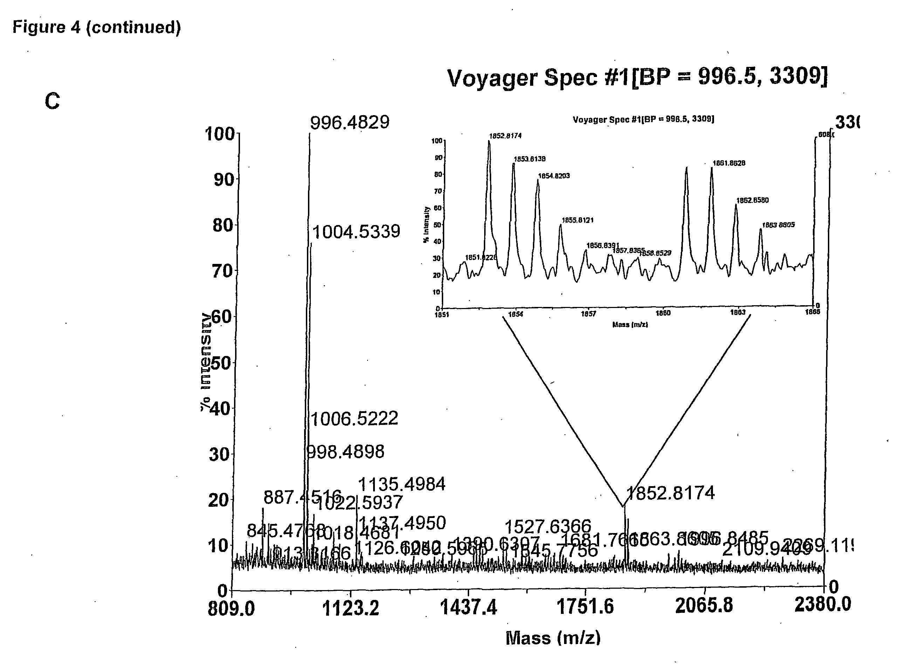

Identification and quantification of the compounds of interest extracted from the sample can be done according to well-known techniques known in the prior art. For each of the compound classes in question, techniques are described that allow identification and quantification in one step and, moreover, are suited to record the respective compounds contained in the extracts in a comprehensive manner.
For example, metabolites may be identified and quantified using gas chromatography/mass spectrometry (GC/MS), liquid chromatography/mass spectrometry (LC/MS), NMR or FT-IR or combinations thereof. Further useful methods include LC/UV, refractory index determination, the use of radioactivity in connection with suitable methods known to the skilled person, thin layer chromatography (TLC), capillary electrophoresis (CE), CE/UV, CE/laser induced fluorescence (LIF), fluorescence detection, electrochemical detection (i.e. colorimetry), direct injection MS, flow injection MS, MS/MS, MS/MS/MS, and further combinations of MS steps (MS"), fourier transform ion mass spectrometry (FT/MS), and gel permeation chromatography (GPC).
An exemplary non-biased analysis is described in Fiehn et al. (2000). With the aim of functionally characterizing plant mutants, detection and relative quantifications of 326 distinct compounds (ranging from primary polar metabolites to sterols) was carried out for both identified and non-identified compounds, after normalization to internal references and plant tissue fresh weights. Different plant mutants were compared to the corresponding parental genotypic backgrounds, and the data were used for statistical analysis as well as for defining metabolic phenotypes that were derived from clustering tools. Another example of GC/MS analyses that can be applied in the method of the invention has been described by Roessner et al. (2001), who used it for comprehensively studying the metabolism in potato tubers. Alternatively, metabolite data can be obtained by extended chromatographic analysis as described by Tweeddale et al. (1998) where, after growing wild type and mutant E.coli strains in minimal media and ¹⁴C-labelled glucose, 70 metabolites could be separated using two dimensional thin layer chromatography. The relative quantification of metabolites was carried out by radioactive detection.
Suitable techniques for identifying and quantifying proteins are known to the person skilled in the art and described in the literature. They include for example LC/MS and two-dimensional electrophoresis and protein staining. A comprehensive overview of different strategies for protein extraction and proteome analysis from various tissue that may be useful in connection with the method of the invention can be found in "2D-Proteome Analysis Protocol" by Andrew Link (1999). For example, the use of 2D-gels combined with mass spectrometry, usually MALDI-TOF, allows the detection and identification of a large number of proteins from a sample in a quality that facilitates the comparison of different protein profiles (Nock et al. 1998; Eggeling et al. 1998). For applications that require a unbiasedness, resolution and/or reproducibility that exceeds the potential of two-dimensional gel electrophoresis (2DE) (e.g. as discussed in Gygi et al. 2000), there are altemative techniques available in the prior art. For instance, Wall et al. have shown that protein mixtures can be fractionated using isoelectric focusing in the liquid phase (Wall et al. 2000, 2001). By coupling to monolithic columns and digesting the protein prior to mass spectrometry, it is possible to achieve the identification of hundreds of proteins. For the purpose of high throughput approaches relative protein quantification as described by Gygi et al. (1999b) may be the technique of choice. When analyzing proteins from yeast, Gygi and co-workers were able to achieve a repeatability better than 12% relative standard deviation. This method involves linking protein Cys-residues to stable isotopically labeled chemicals that include biotin moieties for sample purification and preconcentration. This method, also referred to as isotope-coded affinity tag (ICAT), has been successfully applied in the experiments made in connection with the present invention (see Example and Figures 4B and C). Protein identification and quantification may likewise be done by de novo peptide sequencing (Goodlett et al. 2001). In addition, when applying LC/MS-based methods it may be beneficial to increase the run times of liquid chromatography in order to ensure that only a few peptides per time interval reach the mass spectrometer (Washbum et al. 2001). This may be achieved by stepwise elution of the peptide mixture from strong cation exchangers onto nanoscale reverse phase columns prior to ion trap MS/MS experiments. By applying this strategy, it was possible to reach a genome coverage of ∼25 percent for yeast. In total, 1500 proteins were detected, ranging from low abundant transcription factors to proteins with up to 14 transmembrane domains, and high and low abundant enzymes. Most importantly, it could be proven that no bias against codon usage was found in the detected proteins. With regard to MS and subsequent MS/MS experiments, it may be recommendable to use mass spectrometers with ultimate sensitivity and mass resolution. Here, for example, a combination of nanoLC columns (Li et al. 2001, Shen et al. 2001a, 2001b) or capillary isoelectric focusing (Jensen et al. 2000) prior to fourier-transform ion cyclotron mass spectrometry (FT-MS) can be of use. By applying either of these techniques, up to 10⁶ peptides per run can theoretically be separated, with up to 50,000 peptides found experimentally. Another strategy feasible for the method of the invention may be labeling approaches using stable isotopes. Such a method was originally developed by Aebersold and then adapted to proteomic experiments (Smith et al. 2001, Goshe et al. 2001). Using such a method, it was possible to identify and quantify up to a thousand proteins in 4 h runs. Moreover, by using infrared laser photodissociation, peptides can be fragmented within the cyclotron in order to confirm peptide identification that is for example solely based on accurate masses (Conrads et al. 2000).
RNA can be identified and quantified using a number of techniques currently available in the field such as hybridization on nylonfilters, cDNA microarrays, DNA chips loaded with oligonucleotides (see Granjeaud, Bioessays 21 (1999), 781-790), serial analysis of gene expression (SAGE; Velculescu, Science 270 (1995), 484-487), massively parallel signature sequencing (MPSS; Brenner, Nat. Biotechnol. 18(6) (2000), 630-634) differential-display-based approaches, such as arbitrarily primed (AP) PCR (Welsh, Nucl. Acids Res. 20 (1992), 4965-4970) and cDNA-amplified fragment length polymorphism (AFLP) (Bachem, Plant J. 9 (1996), 745-753). Systematic approaches for a comprehensive mRNA expression profiling that may be applied in connection with the method of the invention are described in Hughes et al. (2000) and Lockhart and Winzeler (2000).

The term "quantitatively analyzing metabolites, proteins and/or RNA" refers to any mathematical analysis method that is suited to further process the quantitative data provided by the method of the invention. This data represents the amount of the compounds analyzed present in each sample either in absolute terms (e.g. weight or moles per weight sample) or in relative terms (i.e. normalized to a certain reference quantity).
Quantitative analysis involves suitable statistical evaluation and correlation analyses. The former includes normalization to the total content of the respective compounds within one compound class, correction of background levels and the combination of the data sets obtained from different compound classes into a single data sheet. Corresponding mathematical methods and computer programs are known to the practicioner. Examples include SAS, SPSS and systatR. As the next step, the statistically pre-treated data may be subjected to a pairwise correlation analysis. Here series of pairs of data points from the analyzed compounds are looked at for correlation, whether positive or negative, for instance using Pearson's correlation coefficient. In a preferred embodiment, the quantitative analysis referred to in the method of the invention furthermore involves network analysis. Network analysis aims at finding out higher order interplays of multiple factors on the basis of pairwise correlation data. By taking several data sets each obtained from one sample, correlations between metabolites, proteins, and/or RNA as well as among these classes of compounds can be analyzed in order to derive information about the network regulation of biological systems, e.g. upon genetic or environmental perturbation. A comprehensive overview of methods for quantitatively analyzing data obtained according to the method of the invention including principle component analysis, "snapshot analysis", pearson correlation analysis, mutual information and network analyses can be found in Fiehn (2001).

The present invention furthermore relates to a method for quantitatively analyzing metabolites, proteins and/or RNA in a biological source material comprising
(a) providing data on metabolites, proteins and/or RNA in said biological source material according to the method described above;
(b) performing suitable statistical evaluation and correlation analysis on the data obtained; and
(c) optionally further performing a network analysis on the data obtained in step (b).
   The features of this method have already been described above in connection with the method for providing data and likewise apply in the context of this aspect of the invention.

In addition, the present invention relates to the use of a mixture of solvents comprising at least a highly polar solvent, at least a less polar solvent and at least a lipophilic solvent as described herein above for extracting metabolites from a sample of a biological source material in order to perform metabolite profiling.
It has been shown that such a mixture of solvents as it is characterized in its various aspects above is better suited for extracting metabolites than other extraction methods described in the prior art such as subsequent application of pure solvents or methanol/water mixtures, or other methods such as microwave extraction, ultrasonication, or accelerated solvent extraction. Thus, it is possible to benefit from the special extraction strength of such a mixture of solvents for metabolite profiling. The term "metabolite profiling" refers to quantitatively analyzing metabolites according to the explanations given above.

According to a preferred embodiment of this use, additionally proteins and/or RNA is extracted from said sample.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.
Furthermore, the term "and/or" when occurring herein includes the meaning of "and", "or" and "all or any other combination of the elements connected by said term".

The present invention is further described by reference to the following non-limiting figures and examples.

### The Figures show:

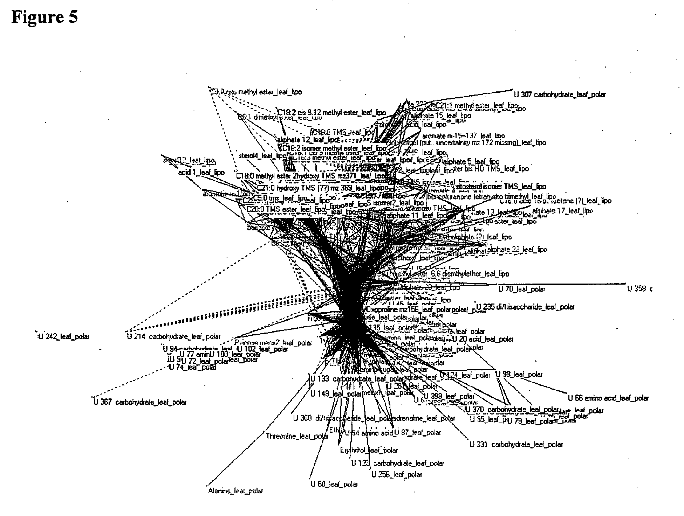

- **Figure 1:**: Scheme illustrating the steps performed in the experiment of the Example.
- **Figure 2:**: A: GC/MS analysis of the metabolites.
B: LC/MS/MS analysis of the metabolites.
- **Figure 3:**: A: SDS PAGE, lanes 1 to 3 show the proteins from three different samples extracted according to the scheme shown in Figure 1.
B: Agarose gel electrophoresis, lines 1 to 9 show RNA from nine different samples extracted according to the scheme shown in Figure 1.
- **Figure 4:**: A: Above: LC/MS/MS analysis of peptides obtained upon trypsination of protein extracted from Arabidopsis leaf according to the method described.
Below: List of proteins identified in the LC/MS/MS analysis.
B and C: ICAT analysis of proteins extracted according to the scheme shown in Figure 1 and explained in the Example from an Arabidopsis sample and an Arabidopsis control sample showing the relative quantification of the proteins (for details see Gygi et al. 1999b). B: list of peptides in one fraction after cation-exchange- and avidin-affinity chromatography. C: MALDI TOF spectrum with enlarged detail of a peptide pair for relative quantification
- **Figure 5:**: Metabolite network of Pearson correlations using 30 samples.

### The following Example illustrates the invention:

### Example: Network analysis of compounds in Arabidopsis leaf

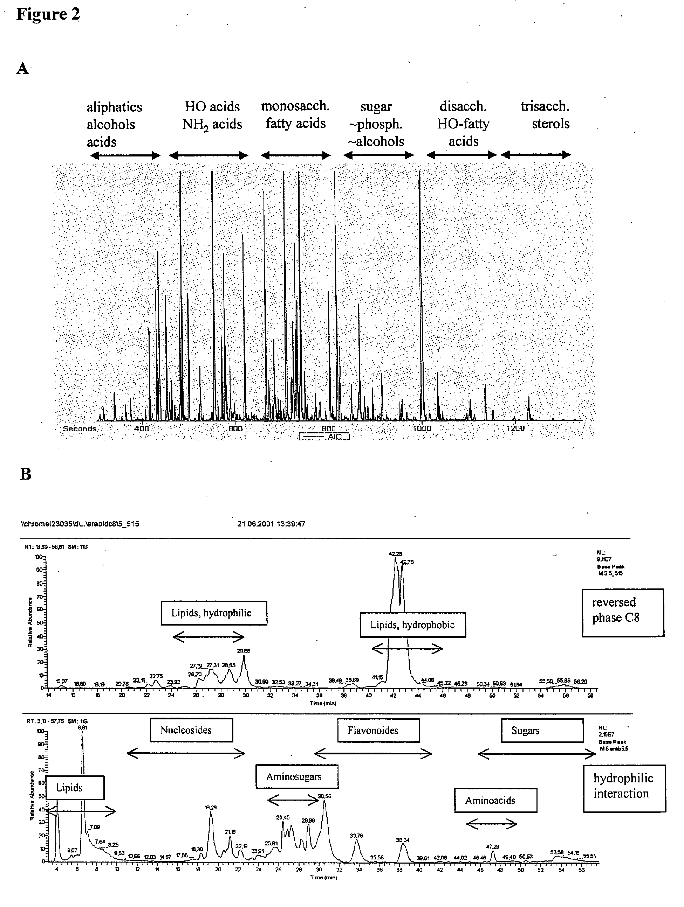

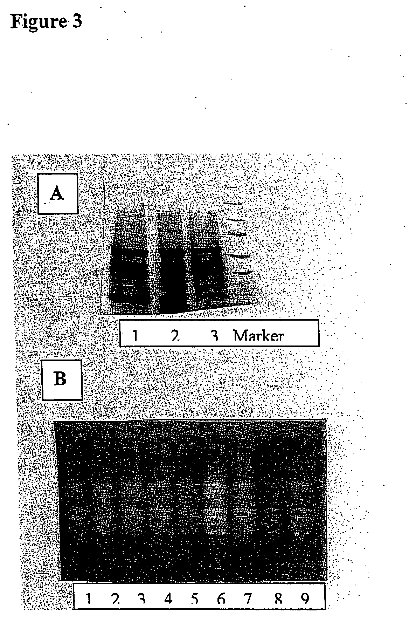

A sample of an *Arabidopsis thaliana* leaf (30mg FW) was extracted with a cold (-16°C) mixture of chloroform / methanol / water (1 : 2.5 : 1 v/v/v). This mixture is a one-phase solution and proved to have an improved extraction strength for metabolites in a comprehensive scale. Identification and relative quantitation of these metabolites was done with GC/MS and LC/MS analysis (see Figure 2A and 2B). In a subsequent step, proteins and RNA were isolated from the remaining cell residue by buffer/phenol extraction and phase separation (see Figure 3A and 3B). The protein was precipitated from the phenol phase with methanol/acetate, washed three times and dried. After tryptic digestion the proteins were identified and relatively quantitated by LC/MS and LC/MS/MS (see Figure 4). RNA was precipitated from the aqueous phase with ethanol and quantitated by gel electrophoresis (see Figure 4).
Last, all data were normalized to the total content of the respective gene products, corrected for background levels, and combined in a single data sheet. For each individual data set, or for the combined data set, network calculation can be done.
As an example, all pair-wise correlations for a metabolite dataset of 30 samples were analyzed for Pearson's correlation coefficients and visualized in a network (see Figure 5).

### References

ap Rees, T. and Hill S.A. 1994. Metabolic control analysis of plant metabolism. *Plant Cell Env.* **17:** 587-599

Benthin, B., Danz, H. and Hamburger, M. 1999. Pressurized liquid extraction of medicinal plants. *J. Chromatogr. A* **837:** 211-219

Blanch, G.P., Caja, M.M., del Castillo, M.L.R., Santa-Mariá, G. and Herraiz, M. 1999. Fractionation of plant extracts by supercritical fluid extraction and direct introduction in capillary gas chromatography using a programmable temperature vaporizer. *J. Chromatogr. Sci.* **37:** 407-410

Bouchereau A., Guénot P. and Larher, F. 2000. Analysis of amines in plant materials. *J. Chromatogr. B* **747:** 49-67

Castioni, P., Christen, P. and Veuthey J.L. 1995. Supercritical fluid extraction of compounds from plant origin. *Analusis* **23:** 95-106

Conrads TP, Anderson GA, Veenstra TD, Pasa-Tolic L, Smith RD (2000) Utility of accurate mass tags for proteome-wide protein identification. Anal. Chem. 72, 3349-3354

Eggeling F V, Roemer, I., Dahse, R., Riese, U., Fiedler, W., Ernst, G., Claussen, U. and Klose, J. (1998) Multicolor two-dimensional gel electrophoresis for mutation detection in cancer related genes. J. Molecular Medicine 76 B48.

Farré, E.M., Tiessen, A., Roessner, U., Geigenberger, P., Trethewey, R.N. and Willmitzer, L. 2001. Analysis of the compartmentation of glycolytic intermediates, nucleotides, sugars, organic acids, amino acids and sugar alcohols in potato tubers using a non-aqueous fractionation method. *Plant Phys.* (*in press)*

Fiehn, O., Kopka, J., Dörmann, P., Altmann, T., Trethewey, R.N. and Willmitzer, L. 2000. Metabolite profiling for plant functional genomics. *Nat. Biotechnol.* **18:** 1157-1161

Fiehn O. (2001) Metabolomics - the link between genotypes and phenotypes. *Plant Mol Biol* (in press)

Gamiz-Gracia, L. and de Castro, M.D.L. 2000. Continuous subcritical water extraction of medicinal plant essential oil: comparison with conventional techniques. *Talanta* **51:** 1179-1185

Gassen, H. G. and Schrimpf, G. (1999) Gentechnische Methoden, Spektrum Akademisher Verlag, Gustav Fischer, Heidelberg Berlin

Gerhardt, R. and Heldt, H.W. 1984. Measurement of subcellular metabolite levels in leaves by fractionation of freeze-stopped material in nonaqueous media. *Plant Physiol.* **75:** 542-547

Gonzalez, B., Francois, J. and Renaud, M. 1997. A rapid and reliable method for metabolite extraction in yeast using boiling buffered ethanol. *Yeast* **13**:1347-1356

Goodlett DR, Keller A, Watts JD, Newitt R, Yi EC, Purvine S, Eng JK, von Haller P, Aebersold R, Kolker E (2001) Differential stable isotope labeling of peptides for quantitation and de novo sequence derivation. Rapid. Commun. Mass Spectrom. 15, 1214-1221

Goshe MB, Conrads TP, Panisko EA, Angell NH, Veenstra TD, Smith RD (2001) Phosphoprotein isotope-coded affinity tag approach for isolating and quantitating phosphopeptides in proteome-wide analyses. Anal. Chem. 73, 2578-2586

Gygi SP, Corthals GL, Zhang Y, Rochon Y, Aebersold R (2000) Evaluation of two-dimensional gel electrophoresis-based proteome analysis technology PNAS 97, 9390-9395

Gygi SP, Rist B, Gerber SA, Turecek F, Gelb MH, Aebersold R (1999b) Quantitative analysis of complex protein mixtures using isotope-coded affinity tags. Nat. Biotechnol.17, 994-999

Gygi SP, Rochon Y, Franza BR, Aebersold R (1999a) Correlation between protein and mRNA abundance in yeast. Mol. Cell Biol. 19, 1720-1730

Hughes TR, Marton MJ, Jones AR, Roberts CJ, Stoughton R, Armour CD, Bennett HA, Coffey E, Dai HY, He YDD, Kidd MJ, King AM, Meyer MR, Slade D, Lum PY, Stepaniants SB, Shoemaker DD, Gachotte D, Chakraburtty K, Simon J, Bard M, Friend SH 2000 Functional discovery via a compendium of expression profiles, **Cell** 102, 109-126

Jarvis, A.P. and Morgan, E.D. 1997. Isolation of plant products by supercritical fluid extraction. *Phytochem. Anal.* **8:** 217-222

Jensen PK, Pasa-Tolic L, Peden KK, Martinovic S, Lipton MS, Anderson GA, Tolic N, Wong KK, Smith RD (2000) Mass spectrometic detection for capillary isoelectric focusing separations of complex protein mixtures. Electrophoresis 21, 1372-1380

Johansen, H.N., Glitso, V. and Knudsen, K.E.B. 1996. Influence of extraction solvent and temperature on the quantitative determination of oligosaccharides from plant materials by high performance liquid chromatography. *J. Agric. Food Chem.* **44**: 1470-1474

Li LJ, Masselon CD, Anderson GA, Pasa-Tolic L, Lee SW, Shen YF, Zhao R, Lipton MS, Conrads TP, Tolic N, Smith RD (2001) High-throughput peptide identification from protein digests using data-dependent multiplexed tandem FTICR mass spectrometry coupled with capillary liquid chromatography. Anal. Chem. 73, 3312-3322

Link, A, J. 1999. 2D Proteome Analysis. In: Methods in Molecular Biology, Volume 112, Humana Press, Totowa, New Jersey

Lockhart DJ, Winzeler EA Genomics, gene expression and DNA arrays 2000 Nature 405, 827-836.

Namiesnik, J. and Gorecki, T. 2000. Sample preparation for chromatographic analysis of plant material. J. *Plan. Chromatogr.* **13**: 404-413

Nock, C., Gauss, C., Himmelbauer, H., Buessow, K., Schalkwyk, L., Loewe, M., Lehrach, H. and Klose J (1998) Connecting the mouse proteome to its genome. J. Molecular Medicine 76 B46.

Roberts, J.K.M. 2000. NMR adventures in the metabolic labyrinth within plants. Trends Plant Sci. 5: 30-34

Roessner, U., Luedemann, A., Brust, D., Fiehn, O., Linke, T., Willmitzer, L. and Femie, A.R. 2001. Metabolic profiling allows comprehensive phenotyping of genetically or environmentally modified plant systems. *Plant Cell* **13:**11-29

Sambrook and Russell (2001) Molecular Cloning: A Laboratory Manual, Cold Spring Harbour Laboratory Press, New York

Sargenti, S.R. and Vichnewski, W. 2000. Sonication and liquid chromatography as a rapid technique for extraction and fractionation of plant material. *Phytochem. Anal.* 11: 69-73

Shen YF, Tolic N, Zhao R, Pasa-Tolic L, Li LJ, Berger SJ, Harkewicz R, Anderson GA, Belov ME, Smith RD (2001a) High-throughput proteomics using high efficiency multiple-capillary liquid chromatography with on-line high-performance ESI FTICR mass spectrometry. Anal. Chem. 73, 3011-3021

Shen YF, Zhao R, Belov ME, Conrads TP, Anderson GA, Tang KQ, Pasa-Tolic L, Veenstra TD, Lipton MS, Udseth HR, Smith RD (2001b) Packed capillary reversed-phase liquid chromatography with high-performance electrospray ionization Fourier transform ion cyclotron resonance mass spectrometry for proteomics. Anal. Chem. 73,1766-1775

Smith RD, Pasa-Tolic L, Lipton MS, Jensen PK, Anderson GA, Shen YF, Conrads TP, Udseth HR, Harkewicz R, Belov ME, Masselon C, Veenstra TD (2001) Rapid quantitative measurements of proteomes by Fourier transform ion cyclotron resonance mass spectrometry. Electrophoresis 22,1652-1668

Starmans, D.A.J. and Nijhus H.H. 1996. Extraction of secondary metabolites from plant material: a review. *Trends Food Sci. Technol.* **7**: 191-197

Streeter J.G. and Strimbu C.E. 1998. Simultaneous extraction and derivatization of carbohydrates from green plant tissues for analysis by gas-liquid chromatography. *Anal. Biochem.* **259:** 253-257

Tweeddale, H., Notley-McRobb, L. and Ferenci, T. 1998. Effect of slow growth on metabolism of Escherichia coli, as revealed by global metabolite pool ("Metabolome") analysis. *J. Bacteriol.* **180:** 5109-5116

Wall DB, Kachman MT, Gong SY, Hinderer R, Parus S, Misek DE, Hanash SM, Lubman DM (2000) Isoelectric focusing nonporous RP HPLC: A two-dimensional liquid-phase separation method for mapping of cellular proteins with identification using MALDI-TOF mass spectrometry. Anal. Chem. 72, 1099-1111

Wall DB, Kachmann MT, Gong SY, Parus SJ, Long, MW, Lubman DM (2001) Isoelectric focusing nonporous silica RP HPLC/ESI TOF MS: a three dimensional liquid-phase protein separation method as applied to human erythroleukemia cell-line. Rapid Commun. Mass Spectrom. 18, 1649-1661

Washburn MP, Wolters D, Yates JR (2001) Large-scale analysis of the yeast proteome by multidimensional protein identification technology. Nat. Biotechnol. 19, 242-247

## Claims

1. A method for providing data useful for quantitatively analyzing metabolites, proteins and/or RNA in a biological source material, whereby said analysis involves suitable statistical evaluation and correlation analysis on the data obtained,
said method being **characterized by** the step of extracting, identifying and quantifying at least two compound classes of the group consisting of metabolites, proteins and RNA from at least one sample from said biological source material, wherein the compounds of said at least two classes are each determined from one sample.

2. The method of claim 1, wherein said analysis furthermore involves network analysis.

3. The method of claim 1 or 2, wherein extracting, identifying and quantifying is carried out taking a multitude of samples.

4. The method of any on of claims 1 to 3, wherein said extracting comprises the steps of:
(a) extracting the metabolites from said sample with at least one solvent or mixture of solvents;
(b) extracting the proteins from the remainder of the sample after step (a);
(c) extracting the RNA from the remainder of the sample after step (a); and
(d) optionally dissolving remaining cellular material contained in said sample.

5. The method of claim 4, wherein said mixture of solvents comprises at least one highly polar solvent, at least one less polar solvent and at least one lipophilic solvent.

6. The method of claim 5, wherein said mixture of solvents comprises water, methanol and chloroform.

7. The method of claim 6, wherein said mixture of solvents contains water, methanol and chloroform in the approximate proportion by volume of 1: 2.5: 1.

8. The method of any one of claims 4 to 7, wherein step (a) is carried out at a temperature between -60 and +4°C.

9. A method for quantitatively analyzing metabolites, proteins and/or RNA in a biological source material comprising
(a) providing data on metabolites, proteins and/or RNA in said biological source material according to the method of any one of claims 1 to 8;
(b) performing suitable statistical evaluation and correlation analysis on the data obtained; and
(c) optionally further performing a network analysis on the data obtained in step (b).

10. Use of a mixture of solvents as defined in any one of claims 4 to 8 for extracting metabolites from a sample of a biological source material in order to perform metabolite profiling.

11. The use of claim 10, wherein additionally proteins and/or RNA is extracted from said sample.
